# EUROPÄISCHE PATENTSCHRIFT

(11) **EP 1 901 860 B1**
(45) Veröffentlichungstag und Bekanntmachung des Hinweises auf die Patenterteilung: **11.01.2012**
(21) Anmeldenummer: 06761718.3
(22) Anmeldetag: 28.06.2006
(51) Int. Cl.: B01D 61/08, B01D 61/18, B01D 65/02, C02F 1/00, A61L 2/22, A61L 2/18

(54) **VORRICHTUNG UND VERFAHREN ZUR REINIGUNG UND/ODER DESINFEKTION VON FILTERN**
DEVICE AND METHOD FOR CLEANING AND/OR DISINFECTING FILTERS
DISPOSITIF ET PROCEDE DE NETTOYAGE ET / OU DE DESINFECTION DE FILTRES

(30) Priorität: 01.07.2005 DE 202005010525 U
(43) Veröffentlichungstag der Anmeldung: 26.03.2008
(73) Patentinhaber: Aqua Carat GmbH, 72622 Nürtingen (DE)
(72) Erfinder: DOPSLAFF, Reinhard, 71364 Winnenden-Birkmannsweiler (DE); DOPSLAFF, Hartmut, 71364 Winnenden (DE); SÖCKNICK, Ralf, 70806 kornwestheim (DE); NEIDHARDT, Klaus, 75228 Ispringen (DE); HESS, Klaus, 73663 Berglen-Reichenbach (DE)
(74) Vertreter: Kohler Schmid Möbus
(86) Internationale Anmeldenummer: PCT/DE2006/001111
(87) Internationale Veröffentlichungsnummer: WO 2007/003164

(56) Entgegenhaltungen:
- EP-A- 0 526 372
- DE-A1- 10 115 633
- DE-A1-102004 048 333
- DE-U1-202004 004 153
- US-A- 5 244 579

## Beschreibung

Die Erfindung betrifft eine Vorrichtung zur Reinigung und/oder Desinfektion von Filtern, die ein Gehäuse mit einem ersten Einlass für zu filtrierendes Wasser und einem Auslass für filtriertes Wasser, eine im Gehäuse befindliche Filtereinheit sowie einen zweiten Einlass für Reinigungs- und/oder Desinfektionsmittel umfasst, und ein Verfahren zur Reinigung und/oder Desinfektion von Filtern mittels einer solchen Vorrichtung.

Filter werden zur Wasseraufbereitung in vielfältiger Weise eingesetzt. DE 20 2004 004 153 U1 beschreibt eine Duschvorrichtung, bei der zur Wasseraufbereitung in der Zuführleitung ein Legionellenfilter angeordnet ist.

DE 101 15 633 A1 beschreibt eine Vorrichtung zur Gewinnung von im Wesentlichen keimfreiem Wasser mittels eines Hohlfasermoduls. Der Filter wirkt als Dead-End-Filter. Aufgrund der geringen Porenweite der Hohlfasern verblockt der Filter sehr rasch und muss regelmäßig getauscht werden. Dies ist vor allem bei teuren Hohlfasermodulen und teuren Membranen von Nachteil. Darüber hinaus besteht beim Filterwechsel die Gefahr der Verschmutzung und Verkeimung des Filtrats, wenn Austauschfiltereinsätze nicht einzeln und hygienisch abgepackt sind oder die für das Auswechseln notwendigen Werkzeuge mit dem Filtrat in Berührung kommen.

DE 199 18 221 C1 und DE 199 61 406 C1 beschreiben Filter, die im cross-flow-Verfahren betrieben werden bzw. rückspülbar sind. Dadurch können Membranen und Filtersiebe mechanisch von Zeit zu Zeit abgereinigt werden. Die Standzeiten verlängern sich erheblich. EP 0 526 372 A1 verwendet zusätzlich noch Luft, die 1:1 mit dem zum Spülen verwendeten Wasser gemischt wird.

Zur Unterstützung des mechanischen Reinigungsvorgangs wird in DE 197 03 877 C1 eine auf der Anströmseite der Filtermembran gebildete Deckschicht durch Aufsprühen von Druckluft oder Filtrat von der Anströmseite der Filtermembran lokal in Richtung auf die Deckschicht mittels einer beweglichen Spüldüse abgereinigt.

In DE 197 50 799 C1 leitet man zum periodischen Ablösen von an der Anströmseite haftenden Ablagerungen einen oder mehrere Gasstöße (Pulsgas) aus einem Druckbehälter durch eine Leitung in den Kanal mit gereinigtem Medium und in die Filterelemente.

Eine rein mechanische Abreinigung der Filteroberflächen ist jedoch oft nicht ausreichend. Besonders bei feinporigen Membranen können hartnäckige, die Poren verstopfende Ablagerungen nicht genügend entfernt werden.

In US 5,244,579, DE 44 45 682 C2 und DE 696 04 818 T2 werden verschiedene Verfahren beschrieben, bei denen Membranen und keramische Filter zusätzlich mit Hilfe von Chemikalienlösungen gereinigt werden.

Bei einem dort erwähnten Verfahren werden die Chemikalien aus einem Vorratsbehälter mit einer Pumpe durch eine separate Leitung zum Filter gefördert. Dies erfordert einen großen apparativen Aufwand. Außerdem ist die Befüllung des Vorratsbehälters mit Chemikalien mit Gefahren verbunden.Bei anderen Verfahren wird das Filtermodul aus dem Gehäuse entnommen und in einer besonders dafür vorgesehenen Vorrichtung gereinigt, oder das Gefäß, in dem die Membranen angeordnet sind, wird vor deren chemischen Reinigung zumindest teilweise entleert. Diese Verfahren sind sehr umständlich und führen zu einem längeren Stillstand der Anlage.

Aufgabe der vorliegenden Erfindung ist daher, oben genannte Nachteile zu vermeiden und eine Vorrichtung und ein Verfahren der eingangs genannten Art vorzustellen, mit denen Filtereinheiten durch einfache und sichere Zugabe von Chemikalien aus einem Druckbehälter effektiv abgereinigt und desinfiziert werden können, ohne dass hierzu ein größerer apparativer Aufbau mit Pumpen, Vorratsgefäßen und Zuleitungen notwendig ist.

Diese Aufgabe wird erfindungsgemäß auf überraschend einfache, aber wirkungsvolle Weise dadurch gelöst, dass die Vorrichtung einen Druckbehälter mit Reinigungs- und/oder Desinfektionsmittel umfasst, dessen Auslassöffnung ohne Zuführleitung mit dem zweiten Einlass des Filtergehäuses für Reinigungs- und/oder Desinfektionsmittel fluiddicht lösbar verbunden ist.

Durch den im Druckbehälter vorliegenden Druck ist es nicht notwendig, die zur Reinigung und/oder Desinfektion der Filtereinheit erforderlichen Chemikalien über ein aufwendiges und teures Pumpensystem zur Filtereinheit zu transportieren. Der direkte Anschluss des Druckbehälters an den zweiten Einlass für Reinigungs- und/oder Desinfektionsmittel ohne Zuführleitung bzw. Schlauchverbindung ermöglicht eine sichere und schnelle Durchführung der Reinigung und/oder Desinfektion der Filtereinheit und damit kurze Stillstandzeiten.

In einer bevorzugten Ausführungsform ist der Druckbehälter eine Sprühdose.

Vorteilhaft sind fertig konfektionierte, handelsübliche Sprühdosen als Druckbehälter. Durch die fertige Konfektionierung in der Sprühdose entfällt der direkte Kontakt mit den Chemikalien. Zudem sind solche Sprühdosen einfach und billig in ihrer Herstellung.

Als umweltfreundliches Treibmittel kann Druckluft verwendet werden. Dabei kann das Treibmittel zusammen mit den Reinigungs- und/oder Desinfektionsmitteln in den Raum um die Filtereinheit eingespeist werden. Die durch das gasförmige Treibmittel entstehenden Verwirbelungen an der Filteroberfläche sorgen für einen zusätzlichen Reinigungseffekt.

Es ist jedoch ebenso möglich, dass die Reinigungs- und/oder Desinfektionsmittel vom gasförmigen Treibmittel im Druckbehälter getrennt sind. Dadurch werden nur die Reinigungs- und/oder Desinfektionsmittel selbst, nicht aber das gasförmige Treibmittel der Filtereinheit zugeführt. Dies ist vorteilhaft, wenn der Druckbehälter der Sprühdose gegen das Reinigungsmittel bzw. die Filtereinheit gegenüber dem Treibmittel chemisch nicht beständig sind.

Bei einer weiteren bevorzugten Ausführungsform beträgt das Volumen des Druckbehälters zwischen 50 ml und 1000 ml, vorzugsweise 100 ml bis 500 ml. Druckbehälter mit derartigen Volumina sind als handelsübliche Sprühdosen billig erhältlich. Zudem sind Druckbehälter in dieser Größe sehr handlich.

Bei einer vorteilhaften Ausführungsform ist ein Endfülldruck im Druckbehälter nach dem vollständigen Einbringen der Reinigungs- und/oder Desinfektionsmittel größer als der Fließdruck im Filtergehäuse.

Dadurch können die Chemikalien zur Reinigung und/oder Desinfektion ohne Pumpensystem in das Filtergehäuse eingespeist werden bis zur vollständigen Entleerung des Druckbehälters. Der Chemikalien können dem Druckbehälter portionsweise bis zur vollständigen Entleerung für mehrere Reinigungen und/oder Desinfektionen entnommen werden. Es ist auch denkbar, dass die Chemikalien für eine Reinigung und/oder Desinfektion fertig konfektioniert sind und damit die für eine Reinigung und/oder Desinfektion der Filtereinheit verbrauchte Menge an Chemikalien genau definiert ist. Durch die vollständige Entleerung des Druckbehälters müssen keine Chemikalienreste entsorgt werden. Der Fließdruck im Filtergehäuse hängt davon ab, ob die Filtereinheit durchströmt, überströmt (cross-flow) oder rückgespült werden soll.

Bei einer besonders vorteilhaften Ausführungsform ist ein Befüllstutzen als Dichtung zwischen der Auslassöffnung des Druckbehälters und dem zweiten Einlass des Filtergehäuses für Reinigungs- und/oder Desinfektionsmittel vorgesehen.

Bei handelsüblichen Sprühdosen befindet sich der Befüllstutzen am Sprühventil und kann direkt in eine sich am Filtergehäuse befindliche, passende Ventilöffnung gesteckt werden. Dadurch öffnet das Rückschlagventil der Sprühdose automatisch und deren Inhalt fließt in das Filtersystem. Es ist somit möglich, Chemikalien auf sichere und einfache Weise dem Filtersystem zuzuführen. Die Gefahr einer Verschmutzung der Umgebung mit Chemikalien ist nahezu ausgeschlossen.

Alternativ befindet sich an der Auslassöffnung des Druckbehälters ein Gewinde, das fluiddicht mit dem zweiten Einlass für Reinigungs- und/oder Desinfektionsmittel am Filtergehäuse verschraubt werden kann. Dadurch ist der Anschluss des Druckbehälters ebenfalls einfach und ohne Werkzeug durchführbar.

Bei einer weiteren Ausführungsform ist im zweiten Einlass für Reinigungs- und/oder Desinfektionsmittel ein Rückflussverhinderer angeordnet. Dadurch wird verhindert, dass durch diesen Einlass Wasser austreten kann.

Bei einer vorteilhaften Variante ist im zweiten Einlass für Reinigungs- und/oder Desinfektionsmittel ein Zeitglied angeordnet.

Reinigungen und/oder Desinfektionen müssen in regelmäßigen Abständen durchgeführt werden. Das Zeitglied erfasst den Moment, in dem der Druckbehälter mit dem Filtergehäuse verbunden wird und die Reinigung und/oder Desinfektion beginnt.

Bei einer vorteilhaften Ausführungsform der Erfindung ist der zweite Einlass für Reinigungs- und/oder Desinfektionsmittel mit der Rohwasserseite verbunden.

Das Reinigungs- und/oder Desinfektionsmittel fließt dann von der Rohwasserseite durch die Filtereinheit auf die Reinwasserseite in den zweiten Auslass für filtriertes Wasser und löst dabei auf der Filteroberfläche haftende und in den Filterporen sich befindliche Ablagerungen. Gleichzeitig wird die Filtereinheit, die komplette Rohwasserseite mit Gehäuse und Filterkuchen sowie die Reinwasserseite mit dem zweiten Auslass für filtriertes Wasser und einem an der Wasseraustrittsstelle angeordneten Perlator desinfiziert. Dies ist besonders wichtig, weil der Bereich um den Perlator durch Spritzwasser oder Kontakt mit schmutzigen Händen leicht verkeimen kann.

Befindet sich auf der Rohwasserseite ein separater Spülkanal, so kann das Reinigungs- und oder Desinfektionsmittel über diesen abfließen. Beim rohwasserseitigen Überströmen der Filtereinheit (cross-flow-Betrieb) werden hohe Strömungsgeschwindigkeiten an der Filteroberfläche erzeugt, die den Reinigungseffekt verstärken. Die Chemikalien werden anschließend gründlich ausgespült.

Dies kann noch dadurch verbessert werden, dass der zweite Einlass für Reinigungs- und/oder Desinfektionsmittel in den ersten Einlass für zu filtrierendes Wasser mündet.

Bei einer alternativen Ausführungsform ist der zweite Einlass für Reinigungs- und/oder Desinfektionsmittel mit der Filtratseite verbunden.

Das Reinigungs- und/oder Desinfektionsmittel fließt dann in umgekehrter Richtung von der Reinwasserseite durch die Filtereinheit in den separaten Spülkanal (Rückspülbetrieb). Durch die chemische Rückspülung werden auf der Filteroberfläche haftende und in den Filterporen sich befindliche Schmutzpartikel effektiv gelöst bzw. abgelöst und ausgespült.

Eine vorteilhafte Weiterbildung dieser Ausführungsform sieht vor, dass der zweite Einlass für Reinigungs- und/oder Desinfektionsmittel in den zweiten Auslass für filtriertes Wasser mündet.

Bei einer bevorzugten Ausführungsform ist im zweiten Auslass für filtriertes Wasser ein Ventil angeordnet. Dieses Ventil kann während der Reinigung und/oder Desinfektion der Filtereinheit sowie beim anschließenden Ausspülen der Chemikalien durch den separaten Spülkanal geschlossen werden. Dadurch baut sich ein für den cross-flow-Betrieb und für die Rückspülung notwendiger Druck auf der Reinwasserseite auf. Darüber hinaus wird sichergestellt, dass kein mit Chemikalien belastetes Wasser als Trinkwasser entnommen wird.

Bevorzugt umfasst die Filtereinheit Membranen oder Hohlfasermodule. Membranen und Hohlfasermodule sind je nach Anwendungsfall in vielen Varianten erhältlich und werden zur Mikro-, Ultra- oder Nanofiltration eingesetzt und dienen zur Filtration, Entkeimung bzw. zur Entsalzung.

Die Filtereinheit kann innerhalb des Gehäuses einer Sanitärarmatur, insbesondere eines Wasserhahnes oder eines Brausekopfes, angeordnet sein. Denkbar wäre beispielsweise, dass die Filtereinheit im Wasserauslauf nach der Ventileinheit der Armatur platziert ist. An dieser Stelle wird nicht nur das Heißwasser, sondern auch Kaltwasser und warmes Mischwasser filtriert.

Alternativ kann die Filtereinheit in einem separaten Gehäuse angeordnet sein, das mit dem Gehäuse einer Wasserauslaufarmatur, insbesondere eines Wasserhahnes oder eines Brausekopfes, verbunden ist. Ebenso kann das Gehäuse der Filtereinheit in einer Zulaufleitung zu einer Wasserauslaufarmatur angeordnet sein. Als Beispiel sei eine Untertisch-Filteranlage genannt.

Bei einer Ausführungsform umfassen die Reinigungsmittel mindestens einen Stoff aus den folgenden Stoffgruppen: Säuren, alkalisierende Stoffe, Tenside, Dispergiermittel, Komplexbildner, Enzyme.

Reinigungsmittel dienen zur Entfernung von Ablagerungen auf der Filteroberfläche und aus Filterporen. Säuren wie z.B. Zitronensäure lösen unter anderem Kalkablagerungen besonders gut, alkalische Reiniger werden hauptsächlich zur Entfernung von organischem und biologischem Fouling verwendet, Tenside und Dispergiermittel wie z.B. Natriumlaurylsulfat setzen die Grenzflächenspannung herab und bewirken eine bessere Benetzung der Schmutzpartikel mit Reinigungslösung, Komplexbildner wie z.B. Zitronensäure oder EDTA verbessern die Membranreinigung durch Entfernung von divalenten Ionen wie Calcium, und Enzyme schließlich können Fette und Proteine spalten.

Bei einer Ausführungsform umfassen die Desinfektionsmittel mindestens einen Stoff aus den folgenden Stoffgruppen: Chlorverbindungen wie Hypochloritlösung oder Chlordioxidlösung, Ozon, Alkohole, Peroxoverbindungen wie Wasserstoffperoxidlösung oder Peressigsäurelösung.

Desinfektionsmittel dienen zur Desinfektion der Rohwasserseite mit Gehäuse und Filterkuchen, der Filtereinheit sowie der Reinwasserseite mit Auslass und Perlator.

Bei einer vorteilhaften Ausführungsform enthalten die Reinigungs- und oder Desinfektionsmittel zusätzlich einen Farbindikator. Das Reinigungs- und/oder Desinfektionsmittel kann dann zum Beispiel mindestens so lange zudosiert werden, bis es an einem Auslass wieder austritt. Der anschließende Waschgang nach der chemischen Reinigung und/oder Desinfektion kann dann so lange durchgeführt werden, bis kein Farbindikator und damit kein Reinigungs- und/oder Desinfektionsmittel mehr austritt.

Bei einer weiteren Ausführungsform ist ein Adapter zur schlauchlosen Verbindung des Druckbehälters mit dem zweiten Einlass für Reinigungs- und/oder Desinfektionsmittel vorgesehen.

Ein Adapter zur schlauchlosen Verbindung eines Druckbehälters an einen Warmwasserkreislauf ist beispielsweise in DE 20 2004 015 423 U1 beschrieben. Dadurch ist es möglich, einen Druckbehälter, dessen Auslassöffnung nicht direkt mit dem Einlass am Filtergehäuse fluiddicht verbindbar ist, ohne Zuführleitung bzw. Schlauchverbindung an ein Filtergehäuse anzuschließen.

In den Rahmen der vorliegenden Erfindung fällt auch ein Verfahren zur Reinigung und/oder Desinfektion von Filtern mit der oben beschriebenen Vorrichtung, bei dem die Auslassöffnung des Druckbehälters mit Reinigungs- und/oder Desinfektionsmittel ohne Zuführleitung mit dem zweiten Einlass für Reinigungs- und/oder Desinfektionsmittel verbunden wird und die in dem Druckbehälter befindlichen Reinigungs- und/oder Desinfektionsmittel nach dem Verbinden in das Gehäuse mit der Filtereinheit eingespeist werden. Hierdurch kann auf ein aufwendiges Pumpen- und Leitungssystem verzichtet werden. Der Umgang mit den zur Reinigung und/oder Desinfektion des Filters notwendigen Chemikalien ist einfach und sicher.

Eine bevorzugte Variante des erfindungsgemäßen Verfahrens ist dadurch gekennzeichnet, dass der Rückflussverhinderer im zweiten Einlass für Reinigungs- und/oder Desinfektionsmittel beim Verbinden mit der Auslassöffnung des Druckbehälters automatisch öffnet. Die Chemikalien werden dann ohne weitere Arbeitsschritte automatisch eingespeist. Beim Trennen des Druckbehälters vom Filtergehäuse schließt der Rückflussverhinderer, und es kann kein Wasser und keine Chemikalien austreten.

Bei einer weiteren Variante wird das Zeitglied beim Verbinden der Auslassöffnung des Druckbehälters mit dem zweiten Einlass für Reinigungs- und/oder Desinfektionsmittel neu gestartet. Filter müssen regelmäßig gereinigt und/oder desinfiziert werden. Das Zeitglied kann beispielsweise nach Ablauf einer bestimmten Zeit ein optisches und/oder akustisches Signal auslösen, das eine notwendige Reinigung und/oder Desinfektion anzeigt.

Bei einer Variante fließt das Reinigungs- und/oder Desinfektionsmittel und Spülwasser durch den zweiten Auslass für filtriertes Wasser ab. Das Reinigungs- und/oder Desinfektionsmittel durchströmt dabei die Filtereinheit in Filtrationsrichtung. Nach dem Reinigen und/oder Desinfizieren werden die Chemikalien gründlich ausgespült. Diese Variante ist konstruktiv sehr einfach realisierbar.

Bei einer bevorzugten Variante fließt das Reinigungs- und/oder Desinfektionsmittel und Spülwasser durch einen separaten Spülkanal ab. Der Reinigungs- und/oder Desinfektionsvorgang kann bei dieser Variante im cross-flow- oder Rückspülbetrieb durchgeführt werden. Die Reinigung ist besonders effektiv, weil Ablagerungen zusätzlich zur chemischen Reinigung durch die Strömung mitgerissen werden.

Bei einer Weiterbildung dieser Variante ist das Ventil im zweiten Auslass für filtriertes Wasser solange geschlossen, bis das Reinigungs- und/oder Desinfektionsmittel vollständig ausgespült ist. Dadurch baut sich ein für den cross-flow-Betrieb und für die Rückspülung notwendiger Druck auf der Reinwasserseite auf. Außerdem wird vermieden, dass mit Reinigungs- und/oder Desinfektionsmitteln belastetes Trinkwasser gezapft wird.

Bei einer weiteren vorteilhaften Variante wird dem Reinigungs- und/oder Desinfektionsmittel ein Farbindikator zugegeben.

Es kann beispielsweise so lange Reinigungs- und/oder Desinfektionsmittel zudosiert werden, bis es an einer Probestelle sichtbar ist. Der anschließende Waschgang nach der chemischen Reinigung und/oder Desinfektion wird so lange durchgeführt, bis kein Farbindikator mehr sichtbar ist und damit kein Reinigungs- und/oder Desinfektionsmittel mehr vorhanden ist.

Weitere Vorteile der Erfindung ergeben sich aus der Beschreibung und der Zeichnung. Ebenso können die vorstehend genannten und die noch weiter aufgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Die Erfindung ist in der Zeichnung dargestellt und wird anhand zwei Ausführungsbeispiele näher erläutert.

Es zeigen:
Fig. 1a eine perspektivische Ansicht einer Sanitärarmatur mit abmontiertem Filtergehäuse und einem Einlass für Chemikalien im Rohwasserbereich;
Fig. 1b einen schematischen Vertikalschnitt durch Fig. 1a mit Anschluss einer Sprühdose als Druckbehälter an die Füllöffnung;
Fig. 2a eine perspektivische Ansicht einer Sanitärarmatur mit abmontiertem Filtergehäuse und einem Einlass für Chemikalien im Filtratbereich und
Fig. 2b einen schematischen Vertikalschnitt durch Fig. 2a mit Anschluss einer Sprühdose als Druckbehälter an die Füllöffnung.

Die Sanitärarmatur **1; 1'** umfasst einen Grundkörper **2; 2'** mit einem Zulauf für kaltes und heißes Wasser **3; 3'; 4; 4',** einer Kartusche **5; 5'** zum Mischen von Kalt- und Heißwasser, einem Stellhebel **6**; **6'** zur Einstellung der Temperatur und der Durchflussmenge von zu zapfendem Wasser und einem ersten Auslass **7; 7'** für zu filtrierendes Mischwasser, ein Gehäuse **8; 8'** mit einer darin angeordneten Filtereinheit **9, 9'** sowie einem ersten Einlass **10, 10'** für zu filtrierendes Wasser und einem zweiten Auslass **11, 11'** für filtriertes Wasser.

Das zu filtrierende Mischwasser fließt über den ersten Auslass 7, 7' der Kartusche 5, 5' und den ersten Einlass 10, 10' in das Filtergehäuse 8, 8' und durchströmt die im Gehäuse 8, 8' befindliche Filtereinheit 9, 9'. Das Filtrat verlässt über den zweiten Auslass 11, 11' die Armatur.

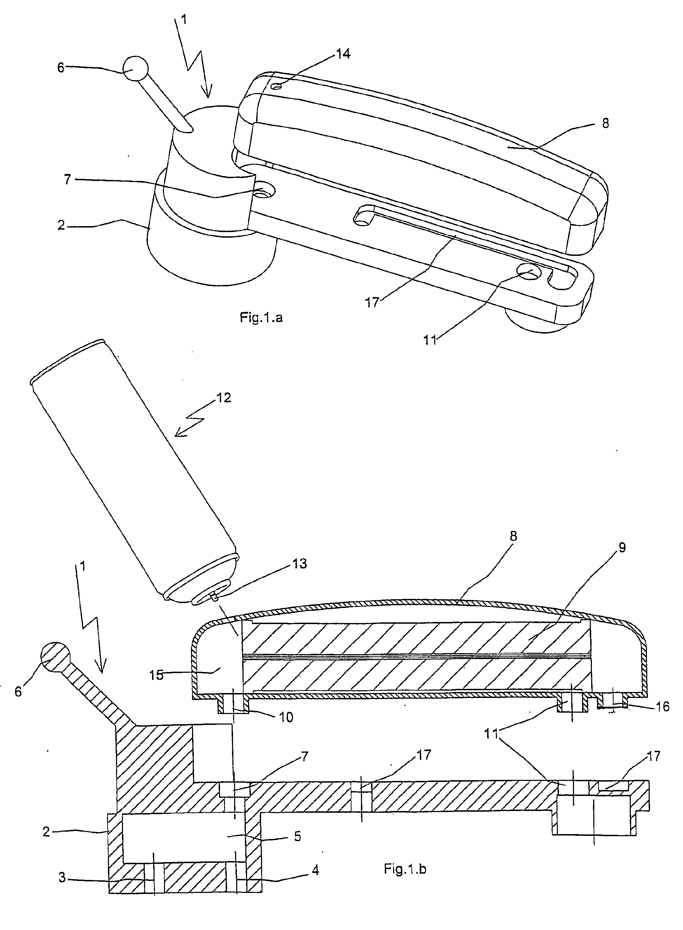

Zur Reinigung und/oder Desinfektion der Filtereinheit 9 wird ein in **Fig. 1b** gezeigter, als Sprühdose ausgelegter Druckbehälter **12** mit Reinigungs- und/oder Desinfektionschemikalien

über einen Befüllstutzen **13** und einen in **Fig. 1a** dargestellten zweiten Einlass **14** fluiddicht auf das Gehäuse 8 aufgedrückt. Dabei öffnet ein in der Abbildung nicht näher dargestelltes Rückschlagventil im zweiten Einlass **14** sowie das in dem Druckbehälter 8 integrierte Rückschlagventil, und die im Druckbehälter 8 befindlichen Chemikalien fließen zur Reinigung und/oder Desinfektion der Filtereinheit 9 in den Rohwasserbereich **15.** Von dort strömt die Reinigungs- und/oder Desinfektionslösung durch die Filtereinheit 9 in den zweiten Auslass 11. Dabei wird die Filtereinheit 9 desinfiziert, Verunreinigungen werden gelöst und ausgeschwemmt. Ein Teil der Reinigungs- und/oder Desinfektionslösung überströmt die Filtereinheit im cross-flow-Verfahren und fließt anschließend über einen dritten Auslass **16** in den Spülkanal **17.** Dabei werden auf der Filteroberfläche haftende Schmutzpartikel gelöst und mit der Strömung mitgerissen. Die Chemikalien werden nach der Reinigung und/oder Desinfektion der Filtereinheit 9 gründlich mit Wasser ausgespült bevor neues Filtrat gezapft werden kann. Ein in der Abbildung nicht dargestelltes, im zweiten Auslass 11 angeordnetes Sicherheitsventil kann während der Reinigung und/oder Desinfektion sowie beim Ausspülen der Chemikalien geschlossen werden, damit kein mit Chemikalien belastetes Wasser als Trinkwasser entnommen werden kann. Die komplette Reinigungs- und/oder Desinfektionslösung fließt dann im cross-flow-Verfahren über die Filteroberfläche in den Spülkanal 17.

Alternativ zur abgebildeten Ausführungsform der Erfindung kann der Spülkanal 17 vollständig innerhalb der Sanitärarmatur 1 verlaufen und durch den Grundkörper 2 abgeführt werden.

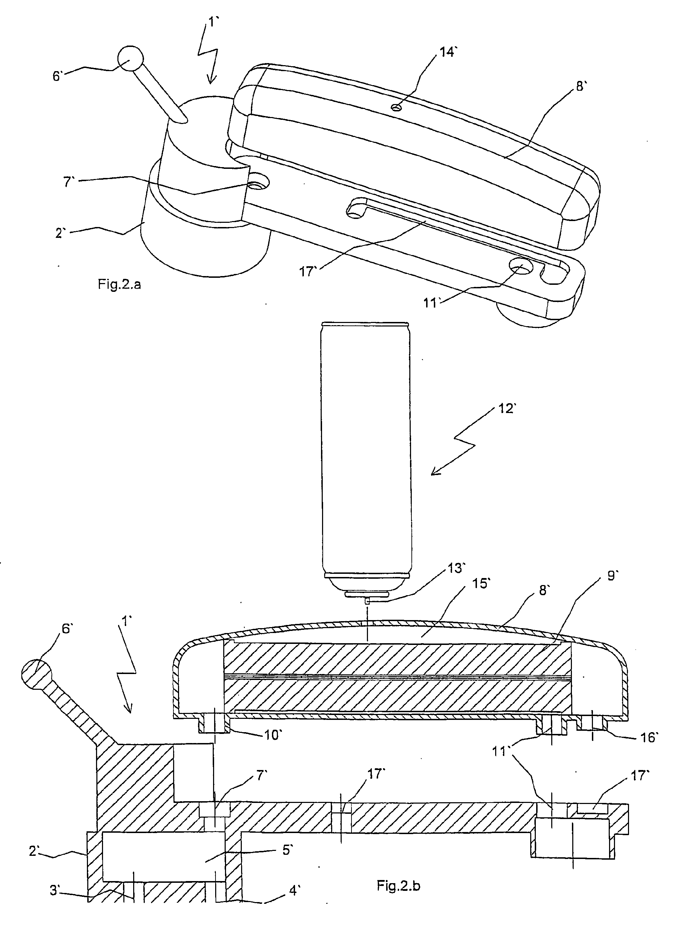

**Fig. 2b** zeigt eine Variante der Erfindung, bei der ein als Sprühdose ausgelegter Druckbehälter **12'** mit Reinigungs- und/oder Desinfektionschemikalien über einen Befüllstutzen **13'** und einen in **Fig. 2a** dargestellten zweiten Einlass **14'** fluiddicht auf das Gehäuse 8' aufgedrückt wird. Der zweite Einlass 14' ist bei dieser Variante direkt mit dem Filtratbereich **15'** verbunden. Ein in der Abbildung nicht dargestelltes, im zweiten Auslass 11' angeordnetes Sicherheitsventil ist dabei geschlossen. Die Reinigungs- und/oder Desinfektionslösung fließt dann in umgekehrter Richtung durch die Filtrationseinheit 9' zur Rohwasserseite und von dort über den dritten Auslass **16'** in den Spülkanal **17'.** Die Filtereinheit wird effektiv gereinigt und desinfiziert durch die Rückspülung mit Reinigungs- und/oder Desinfektionslösung.

### Bezugszeichenliste

- 1; 1': Sanitärarmatur
- 2; 2': Grundkörper
- 3; 3': Zulauf Kaltwasser
- 4; 4': Zulauf Heißwasser
- 5; 5': Kartusche
- 6; 6': Stellhebel
- 7; 7': erster Auslass für zu filtrierendes Mischwasser
- 8; 8': Gehäuse
- 9; 9': Filtereinheit
- 10; 10': erster Einlass für zu filtrierendes Wasser
- 11; 11': zweiter Auslass für filtriertes Wasser
- 12; 12': Druckbehälter
- 13; 13': Befüllstutzen
- 14; 14': zweiter Einlass für Reinigungs- und/oder Desinfektionsmittel
- 15: Rohwasserbereich
- 15': Reinwasserbereich
- 16; 16': dritter Auslass
- 17; 17': Spülkanal

## Patentansprüche

1. Vorrichtung zur Reinigung und/oder Desinfektion von Filtern, die ein Gehäuse (8; 8') mit einem ersten Einlass (10; 10') für zu filtrierendes Wasser und einem zweiten Auslass (11; 11') für filtriertes Wasser, eine im Gehäuse (8; 8') befindliche Filtereinheit (9; 9') sowie einen zweiten Einlass (14; 14') für Reinigungs- und/oder Desinfektionsmittel umfasst,
**dadurch gekennzeichnet,**
**dass** die Vorrichtung einen Druckbehälter (12; 12') mit Reinigungs- und/oder Desinfektionsmittel umfasst, dessen Auslassöffnung ohne Zuführleitung mit dem zweiten Einlass (14; 14') für Reinigungs- und/oder Desinfektionsmittel fluiddicht lösbar verbunden ist.

2. Vorrichtung nach Anspruch 1 **dadurch gekennzeichnet, dass** der Druckbehälter (12; 12') eine Sprühdose ist.

3. Vorrichtung nach einem der Ansprüche 1 oder 2, **dadurch gekennzeichnet, dass** der Druckbehälter (12; 12') ein Volumen zwischen 50 ml und 1000 ml, vorzugsweise 100 ml bis 500 ml aufweist.

4. Vorrichtung nach einem der vorhergehenden Ansprüche, **dadurch gekennzeichnet, dass** ein Endfülldruck im Druckbehälter (12; 12') nach dem vollständigen Einbringen der Reinigungs- und/oder Desinfektionsmittel größer ist als der Fließdruck im Gehäuse (8; 8').

5. Vorrichtung nach einem der vorhergehenden Ansprüche, **dadurch gekennzeichnet, dass** ein Befüllstutzen (13; 13') als Dichtung zwischen der Auslassöffnung des Druckbehälters (12; 12') und dem zweiten Einlass (14; 14') des Gehäuses (8; 8') für Reinigungs- und/oder Desinfektionsmittel vorgesehen ist.

6. Vorrichtung nach einem der vorhergehenden Ansprüche, **dadurch gekennzeichnet, dass** die Auslassöffnung des Druckbehälters (12; 12') ein Gewinde aufweist, das fluiddicht mit dem zweiten Einlass (14; 14') für Reinigungs- und/oder Desinfektionsmittel am Gehäuse (8; 8') verschraubt werden kann.

7. Vorrichtung nach einem der vorhergehenden Ansprüche, **dadurch gekennzeichnet, dass** im zweiten Einlass (14; 14') für Reinigungs- und/oder Desinfektionsmittel ein Rückflussverhinderer angeordnet ist.

8. Vorrichtung nach einem der vorhergehenden Ansprüche, **dadurch gekennzeichnet, dass** im zweiten Einlass (14; 14') für Reinigungs- und/oder Desinfektionsmittel ein Zeitglied angeordnet ist.

9. Vorrichtung nach einem der vorhergehenden Ansprüche, **dadurch gekennzeichnet, dass** der zweite Einlass (14) für Reinigungs- und/oder Desinfektionsmittel mit der Rohwasserseite (15) der Vorrichtung verbunden ist.

10. Vorrichtung nach Anspruch 9, **dadurch gekennzeichnet, dass** der zweite Einlass (14) für Reinigungs- und/oder Desinfektionsmittel in den ersten Einlass (10) für zu filtrierendes Wasser mündet.

11. Vorrichtung nach einem der Ansprüche 1 bis 8, **dadurch gekennzeichnet, dass** der zweite Einlass (14') für Reinigungs- und/oder Desinfektionsmittel mit der Filtratseite (15') der Vorrichtung verbunden ist.

12. Vorrichtung nach Anspruch 11, **dadurch gekennzeichnet, dass** der zweite Einlass (14') für Reinigungs- und/oder Desinfektionsmittel in den zweiten Auslass (11') für filtriertes Wasser mündet.

13. Vorrichtung nach einem der vorhergehenden Ansprüche, **dadurch gekennzeichnet, dass** im zweiten Auslass (11; 11') für filtriertes Wasser ein Ventil angeordnet ist.

14. Vorrichtung nach einem der vorhergehenden Ansprüche, **dadurch gekennzeichnet, dass** die Filtereinheit (9; 9') eine Membran oder ein Hohlfasermodul umfasst.

15. Vorrichtung nach einem der vorhergehenden Ansprüche, **dadurch gekennzeichnet, dass** die Filtereinheit (9; 9') im Gehäuse einer Sanitärarmatur (1; 1'), insbesondere eines Wasserhahnes oder eines Brausekopfes, angeordnet ist.

16. Vorrichtung nach Anspruch 15, **dadurch gekennzeichnet, dass** die Filtereinheit (9; 9) in Fließrichtung nach einer Kartusche (5; 5'), insbesondere einer Ventilkartusche, zum Mischen von Kalt- und Heißwasser angeordnet ist.

17. Vorrichtung nach einem der Ansprüche 1 bis 14, **dadurch gekennzeichnet, dass** das Gehäuse (8; 8') der Filtereinheit (9; 9') mit dem Gehäuse einer Sanitärarmatur (1; 1'), insbesondere eines Wasserhahnes oder eines Brausekopfes, verbindbar ist.

18. Vorrichtung nach einem der Ansprüche 1 bis 14, **dadurch gekennzeichnet, dass** das Gehäuse (8; 8') der Filtereinheit (9; 9') in einer Zulaufleitung zu einer Wasserauslaufarmatur angeordnet ist.

19. Vorrichtung nach einem der Ansprüche 1 bis 14, **dadurch gekennzeichnet, dass** die Filtereinheit (9; 9') als Membran in einer Umkehrosmose-Anlage ausgebildet ist.

20. Vorrichtung nach einem der vorhergehenden Ansprüche, **dadurch gekennzeichnet, dass** die Reinigungsmittel mindestens einen Stoff aus den folgenden Stoffgruppen umfassen: Säuren, alkalisierende Stoffe, Tenside, Dispergiermittel, Komplexbildner, Enzyme.

21. Vorrichtung nach einem der vorhergehenden Ansprüche, **dadurch gekennzeichnet, dass** die Desinfektionsmittel mindestens einen Stoff aus den folgenden Stoffgruppen umfassen: Chlorverbindungen, Ozon, Alkohole, Peroxoverbindungen.

22. Vorrichtung nach einem der vorhergehenden Ansprüche, **dadurch gekennzeichnet, dass** die Reinigungs- und oder Desinfektionsmittel zusätzlich einen Farbindikator enthalten.

23. Vorrichtung nach einem der vorhergehenden Ansprüche, **dadurch gekennzeichnet, dass** ein Adapter zur schlauchlosen Verbindung des Druckbehälters (12; 12') mit dem zweiten Einlass (14; 14') für Reinigungs- und/oder Desinfektionsmittel vorgesehen ist.

24. Verfahren zur Reinigung und/oder Desinfektion von Filtern mittels einer Vorrichtung nach einem der vorhergehenden Ansprüche, **dadurch gekennzeichnet, dass** die Auslassöffnung des Druckbehälters (12; 12') mit Reinigungs- und/oder Desinfektionschemikalien ohne Zuführleitung mit dem zweiten Einlass (14; 14') für Reinigungs- und/oder Desinfektionsmittel verbunden wird und die in dem Druckbehälter (12; 12') befindlichen Reinigungs- und/oder Desinfektionsmittel nach dem Verbinden in das Gehäuse (8; 8') mit der Filtereinheit (9; 9') eingespeist werden.

25. Verfahren nach Anspruch 24 zum Betrieb einer Vorrichtung nach Anspruch 7, **dadurch gekennzeichnet, dass** der Rückflussverhinderer im zweiten Einlass (14; 14') für Reinigungs- und/oder Desinfektionsmittel beim Verbinden mit der Auslassöffnung des Druckbehälters (12; 12') automatisch öffnet.

26. Verfahren nach einem der Ansprüche 24 oder 25 zum Betrieb einer Vorrichtung nach Anspruch 8, **dadurch gekennzeichnet, dass** das Zeitglied beim Verbinden der Auslassöffnung des Druckbehälters (12; 12') mit dem zweiten Einlass (14; 14') für Reinigungs- und/oder Desinfektionsmittel neu gestartet wird.

27. Verfahren nach einem der Ansprüche 24 bis 26, **dadurch gekennzeichnet, dass** das Reinigungs- und/oder Desinfektionsmittel und Spülwasser durch den zweiten Auslass (11; 11') für filtriertes Wasser abfließt.

28. Verfahren nach einem der Ansprüche 24 bis 26, **dadurch gekennzeichnet, dass** ein Ventil im zweiten Auslass (11; 11') für filtriertes Wasser solange geschlossen ist, bis das Reinigungs- und/oder Desinfektionsmittel vollständig ausgespült ist.

29. Verfahren nach Anspruch 28, **dadurch gekennzeichnet, dass** das Reinigungs- und/oder Desinfektionsmittel und Spülwasser durch einen dritten Auslass (16; 16') und einen separaten Spülkanal (17; 17') abfließt.

30. Verfahren nach einem der Ansprüche 24 bis 29, **dadurch gekennzeichnet, dass** dem Reinigungs- und/oder Desinfektionsmittel ein Farbindikator zugegeben wird.

## Claims

1. Device for cleaning and/or disinfecting filters, comprising a housing (8; 8') with a first inlet (10; 10') for water to be filtered, and a second outlet (11; 11') for filtered water, a filter unit (9; 9') located in the housing (8; 8'), and a second inlet (14; 14') for cleaning agents and/or disinfectants,
**characterized in that**
the device comprises a pressure container (12; 12') with cleaning agents and/or disinfectants, the outlet opening of which is detachably connected in a fluid-tight manner to the second inlet (14; 14') for cleaning agents and/or disinfectants without feed line.

2. Device according to claim 1, **characterized in that** the pressure container (12; 12') is a spray can.

3. Device according to one of the claims 1 or 2, **characterized in that** the pressure container (12; 12') has a volume of between 50 ml and 1000 ml, advantageously 100 ml to 500 ml.

4. Device according to any one of the preceding claims, **characterized in that** a final filling pressure in the pressure container (12; 12') after complete introduction of the cleaning agents and/or disinfectants is larger than the flow pressure in the housing (8; 8').

5. Device according to any one of the preceding claims, **characterized in that** a filling nozzle (13; 13') is provided as seal between the outlet opening of the pressure container (12; 12') and the second inlet (14; 14') of the housing (8; 8') for cleaning agents and/or disinfectants.

6. Device according to any one of the preceding claims, **characterized in that** the outlet opening of the pressure container (12; 12') has a thread which can be screwed in a fluid-tight manner to the second inlet (14; 14') for cleaning agents and/or disinfectants provided on the housing (8; 8').

7. Device according to any one of the preceding claims, **characterized in that** a backflow preventer is arranged in the second inlet (14; 14') for cleaning agents and/or disinfectants.

8. Device according to any one of the preceding claims, **characterized in that** a timing element is disposed in the second inlet (14; 14') for cleaning agents and/or disinfectants.

9. Device according to any one of the preceding claims, **characterized in that** the second inlet (14) for cleaning agents and/or disinfectants is connected to the untreated water side (15) of the device.

10. Device according to claim 9, **characterized in that** the second inlet (14) for cleaning agents and/or disinfectants terminates in the first inlet (10) for water to be filtered.

11. Device according to any one of the claims 1 through 8, **characterized in that** the second inlet (14') for cleaning agents and/or disinfectants is connected to the filtrate side (15') of the device.

12. Device according to claim 11, **characterized in that** the second inlet (14') for cleaning agents and/or disinfectants terminates in the second outlet (11') for filtered water.

13. Device according to any one of the preceding claims, **characterized in that** a valve is disposed in the second outlet (11; 11') for filtered water.

14. Device according to any one of the preceding claims, **characterized in that** the filter unit (9; 9') comprises a diaphragm or a hollow fiber module.

15. Device according to any one of the preceding claims, **characterized in that** the filter unit (9; 9') is disposed in the housing of a sanitary fitting (1; 1'), in particular, of a water tap or a shower head.

16. Device according to claim 15, **characterized in that** the filter unit (9; 9') is disposed in the flow direction downstream of a cartridge (5; 5'), in particular a valve cartridge, for mixing cold and hot water.

17. Device according to any one of the claims 1 through 14, **characterized in that** the housing (8; 8') of the filter unit (9; 9') can be connected to the housing of a sanitary fitting (1; 1'), in particular, of a water tap or a shower head.

18. Device according to any one of the claims 1 through 14, **characterized in that** the housing (8; 8') of the filter unit (9; 9') is disposed in a feed line towards a water outlet fitting.

19. Device according to any one of the claims 1 through 14, **characterized in that** the filter unit (9; 9') is designed as a diaphragm in a reverse osmosis system.

20. Device according to any one of the preceding claims, **characterized in that** the cleaning means comprise at least one substance from the following substance groups: acids, alkalizing substances, surfactants, dispersing agents, complexing agents, enzymes.

21. Device according to any one of the preceding claims, **characterized in that** the disinfectants comprise at least one substance from the following substance groups: chlorine compounds, ozone, alcohols, peroxo-compounds.

22. Device according to any one of the preceding claims, **characterized in that** the cleaning agents and/or disinfectants additionally contain a colour indicator.

23. Device according to any one of the preceding claims, **characterized in that** an adaptor is provided for hose-free connection of the pressure container (12; 12') to the second inlet (14; 14') for cleaning agents and/or disinfectants.

24. Method for cleaning and/or disinfecting filters using a device according to any one of the preceding claims, **characterized in that** the outlet opening of the pressure container (12; 12') with cleaning and/or disinfecting chemicals is connected without feed line to the second inlet (14; 14') for cleaning agents and/or disinfectants and the cleaning agents and/or disinfectants contained in the pressure container (12; 12') are fed into the housing (8; 8') with filter unit (9; 9') after connection.

25. Method according to claim 24 for operating a device according to claim 7, **characterized in that** the backflow preventer in the second inlet (14; 14') for cleaning agents and/or disinfectants automatically opens upon connection to the outlet opening of the pressure container (12; 12').

26. Method according to any one of the claims 24 or 25 for operating a device according to claim 8, **characterized in that** the timing element is restarted when connecting the outlet opening of the pressure container (12; 12') to the second inlet (14; 14') for cleaning agents and/or disinfectants.

27. Method according to any one of the claims 24 to 26, **characterized in that** the cleaning agents and/or disinfectants and rinsing water are discharged through the second outlet (11; 11') for filtered water.

28. Method according to any one of the claims 24 to 26, **characterized in that** a valve in the second outlet (11; 11') for filtered water is closed until the cleaning agents and/or disinfectants have been completely rinsed.

29. Method according to claim 28, **characterized in that** the cleaning agents and/or disinfectants and rinsing water are discharged through a third outlet (16; 16') and a separate rinsing channel (17; 17').

30. Method according to any one of the claims 24 through 29, **characterized in that** a colour indicator is added to the cleaning agents and/or disinfectants.

## Revendications

1. Dispositif de nettoyage et/ou de désinfection de filtres, qui comprend un boîtier (8 ; 8') avec une première entrée (10 ; 10') pour l'eau à filtrer et une deuxième sortie (11 ; 11') pour l'eau filtrée, une unité de filtration (9 ; 9') se trouvant dans le boîtier (8 ; 8'), ainsi qu'une seconde entrée (14 ; 14') pour les produits de nettoyage et/ou de désinfection,
**caractérisé en ce que**
le dispositif comprend un récipient sous pression (12 ; 12') avec des produits de nettoyage et/ou de désinfection, dont l'ouverture de sortie est raccordée mobile de manière étanche aux fluides sans conduit d'alimentation à la seconde entrée (14 ; 14') pour les produits de nettoyage et/ou de désinfection.

2. Dispositif selon la revendication 1, **caractérisé en ce que** le récipient sous pression (12 ; 12') est une bombe à aérosol.

3. Dispositif selon l'une quelconque des revendications 1 ou 2, **caractérisé en ce que** le récipient sous pression (12 ; 12') présente un volume compris entre 50 ml et 1000 ml, de préférence de 100 ml à 500 ml.

4. Dispositif selon l'une quelconque des revendications précédentes, **caractérisé en ce qu'**une pression de remplissage finale dans le récipient sous pression (12 ; 12') après l'introduction complète des produits de nettoyage et/ou de désinfection est supérieure à la pression d'écoulement dans le boîtier (8 ; 8').

5. Dispositif selon l'une quelconque des revendications précédentes, **caractérisé en ce qu'**un raccord de remplissage (13 ; 13') est prévu comme joint d'étanchéité entre l'ouverture de sortie du récipient sous pression (12 ; 12') et la seconde entrée (14 ; 14') du boîtier (8 ; 8') pour les produits de nettoyage et/ou de désinfection.

6. Dispositif selon l'une quelconque des revendications précédentes, **caractérisé en ce que** l'ouverture de sortie du récipient sous pression (12 ; 12') présente un filet qui peut être vissé de manière étanche aux fluides sur la seconde entrée (14 ; 14') pour les produits de nettoyage et/ou de désinfection sur le boîtier (8 ; 8").

7. Dispositif selon l'une quelconque des revendications précédentes, **caractérisé en ce qu'**un dispositif anti-reflux est agencé dans la seconde entrée (14 ; 14') pour les produits de nettoyage et/ou désinfection.

8. Dispositif selon l'une quelconque des revendications précédentes, **caractérisé en ce qu'**un élément de temporisation est agencé dans la seconde entrée (14 ; 14') pour les produits de nettoyage et/ou désinfection.

9. Dispositif selon l'une quelconque des revendications précédentes, **caractérisé en ce que** la seconde entrée (14) pour les produits de nettoyage et/ou de désinfection est raccordée au côté eau brute (15) du dispositif.

10. Dispositif selon la revendication 9, **caractérisé en ce que** la seconde entrée (14) pour les produits de nettoyage et/ou de désinfection débouche dans la première entrée (10) pour l'eau à filtrer.

11. Dispositif selon l'une quelconque des revendications 1 à 8, **caractérisé en ce que** la seconde entrée (14') pour les produits de nettoyage et/ou de désinfection est raccordée au côté filtrat (15') du dispositif.

12. Dispositif selon la revendication 11, **caractérisé en ce que** la seconde entrée (14') pour les produits de nettoyage et/ou de désinfection débouche dans la deuxième sortie (11') pour l'eau filtrée.

13. Dispositif selon l'une quelconque des revendications précédentes, **caractérisé en ce qu'**une soupape est agencée dans la deuxième sortie (11 ; 11') pour l'eau filtrée.

14. Dispositif selon l'une quelconque des revendications précédentes, **caractérisé en ce que** l'unité de filtrage (9 ; 9') comprend une membrane ou un module à fibres creuses.

15. Dispositif selon l'une quelconque des revendications précédentes, **caractérisé en ce que** l'unité de filtration (9 ; 9') est agencée dans le boîtier d'une garniture sanitaire (1 ; 1'), en particulier d'un robinet d'eau ou d'une pomme de douche.

16. Dispositif selon la revendication 15, **caractérisé en ce que** l'unité de filtration (9 ; 9) est agencée dans le sens d'écoulement après une cartouche (5 ; 5'), en particulier une cartouche à soupape, pour le mélange d'eau froide et d'eau chaude.

17. Dispositif selon l'une quelconque des revendications 1 à 14, **caractérisé en ce que** le boîtier (8 ; 8') de l'unité de filtration (9 ; 9') peut être raccordé au boîtier d'une garniture sanitaire (1 ; 1'), en particulier d'un robinet d'eau ou d'un pommeau de douche.

18. Dispositif selon l'une quelconque des revendications 1 à 14, **caractérisé en ce que** le boîtier (8 ; 8') de l'unité de filtration (9 ; 9') est agencé dans un conduit d'alimentation menant à une garniture d'évacuation d'eau.

19. Dispositif selon l'une quelconque des revendications 1 à 14, **caractérisé en ce que** l'unité de filtration (9 ; 9') se présente sous la forme d'une membrane dans une installation à osmose inverse.

20. Dispositif selon l'une quelconque des revendications précédentes, **caractérisé en ce que** les produits de nettoyage comprennent au moins une substance choisie parmi les groupes de substances suivants : acides, substances alcalinisantes, agents tensioactifs, agents dispersants, agents complexants, enzymes.

21. Dispositif selon l'une quelconque des revendications précédentes, **caractérisé en ce que** les produits de désinfection comprennent au moins une substance parmi les groupes de substances suivants : composés chlorés, ozone, alcools, composés peroxo.

22. Dispositif selon l'une quelconque des revendications précédentes, **caractérisé en ce que** les produits de nettoyage et/ou de désinfection contiennent en outre un indicateur de couleur.

23. Dispositif selon l'une quelconque des revendications précédentes, **caractérisé en ce que** l'on prévoit un adaptateur pour le raccord sans tuyau du récipient sous pression (12 ; 12') avec la seconde entrée (14 ; 14') pour les produits de nettoyage et/ou de désinfection.

24. Procédé de nettoyage et/ou de désinfection de filtres au moyen d'un dispositif selon l'une quelconque des revendications précédentes, **caractérisé en ce que** l'ouverture de sortie du récipient sous pression (12 ; 12') avec des produits chimiques de nettoyage et/ou de désinfection est raccordée sans conduit d'alimentation à la seconde entrée (14 ; 14') pour les produits de nettoyage et/ou de désinfection et **en ce que** les produits de nettoyage et/ou de désinfection se trouvant dans le récipient sous pression (12 ; 12') sont injectés après le raccordement dans le boîtier (8 ; 8') avec l'unité de filtration (9 ; 9').

25. Procédé selon la revendication 24 pour faire fonctionner un dispositif selon la revendication 7, **caractérisé en ce que** le dispositif anti-reflux s' ouvre automatiquement dans la seconde entrée (14 ; 14') pour les produits de nettoyage et/ou de désinfection lors du raccordement avec l' ouverture de sortie du récipient sous pression (12 ; 12').

26. Procédé selon l'une quelconque des revendications 24 ou 25 pour faire fonctionner un dispositif selon la revendication 8, **caractérisé en ce que** l'élément de temporisation est re-lancé lors du raccordement de l'ouverture de sortie du récipient sous pression (12 ; 12') avec la seconde entrée (14 ; 14') pour les produits de nettoyage et/ou de désinfection.

27. Procédé selon l'une quelconque des revendications 24 à 26, **caractérisé en ce que** le produit de nettoyage et/ou de désinfection et l'eau de rinçage s'écoulent à travers la deuxième sortie (11 ; 11') pour l'eau filtrée.

28. Procédé selon l'une quelconque des revendications 24 à 26, **caractérisé en ce qu'**une soupape est fermée dans la deuxième sortie (11 ; 11') pour l'eau filtrée jusqu'à ce que le produit de nettoyage et/ou de désinfection soit complètement lixivié.

29. Procédé selon la revendication 28, **caractérisé en ce que** le produit de nettoyage et/ou de désinfection et l'eau de rinçage s'évacuent à travers une troisième sortie (16 ; 16') et un canal de rinçage séparé (17 ; 17').

30. Procédé selon l'une quelconque des revendications 24 à 29, **caractérisé en ce qu'**un indicateur de couleur est ajouté au produit de nettoyage et/ou de désinfection.
